# FASCICULE DE BREVET EUROPEEN

(11) **EP 0 905 085 B1**
(45) Date de publication et mention de la délivrance du brevet: **11.04.2007**
(21) Numéro de dépôt: 98203170.0
(22) Date de dépôt: 18.09.1998
(51) Int. Cl.: C01B 7/19, C07C 17/38

(54) **Procédé de séparation de fluorure d'hydrogène de ses mélanges avec un hydrofluoroalcane contenant de 3 à 6 atomes de carbone**
Verfahren zur Abtrennung von Fluorwasserstoff aus seinen Mischungen mit einem Fluorkohlenwasserstoff die 3 bis 6 Kohlenstoffatome enthält
Process for the separation of hydrogen fluoride from its mixtures with a hydrofluoroalkane having 3 to 6 carbon atoms

(30) Priorité: 24.09.1997 FR 9711975
(43) Date de publication de la demande: 31.03.1999
(73) Titulaire: SOLVAY (Société Anonyme), 1050 Bruxelles (BE)
(72) Inventeur: Herkelmann, Ralf, 74906 Bad Rappenau (DE); Brosch, Carsten, 30173 Hannover (DE); Wilmet, Vincent, 1301 Wavre (BE)
(74) Mandataire: Jacques, Philippe

(56) Documents cités:
- EP-A- 0 588 676
- EP-A- 0 601 373
- WO-A-97/13719
- WO-A-97/49656
- DATABASE WPI Section Ch, Week 7502 Derwent Publications Ltd., London, GB; Class E16, AN 75-02816W XP002065362 & JP 49 045842 B (ELECTRO CHEM IND KK)
- PATENT ABSTRACTS OF JAPAN vol. 097, no. 008, 29 août 1997 & JP 09 110738 A (DAIKIN IND LTD), 28 avril 1997

## Description

La présente invention concerne un procédé de séparation de fluorure d'hydrogène de ses mélanges avec un hydrofluoroalcane contenant de 3 à 6 atomes de carbone.

Les hydrofluoroalcanes peuvent être préparés par réaction d'un précurseur chloré approprié avec du fluorure d'hydrogène, tel que décrit par exemple dans les demandes de brevets EP-A1-0699649 et WO-A1-97/15540 (au nom de SOLVAY) et dans la demande de brevet WO-A1-97/05089. Dans un tel procédé, à la sortie du réacteur d'hydrofluoration, le mélange de produits réactionnels contient, outre l'hydrofluoroalcane désiré, du chlorure d'hydrogène provenant de l'élimination du ou des atomes de chlore du précurseur chloré de départ, du fluorure d'hydrogène et du précurseur chloré non transformés, éventuellement des diluants inertes, ainsi que divers sous-produits en faibles quantités. Etant donné que l'on travaille habituellement avec un excès de fluorure d'hydrogène par rapport au précurseur chloré, il subsiste le plus souvent du fluorure d'hydrogène non converti dans le mélange de produits réactionnels. Alors que la plupart des constituants du mélange de produits réactionnels peuvent être facilement séparés complètement par distillation, une séparation complète entre le fluorure d'hydrogène et l'hydrofluoroalcane est généralement très difficilement réalisable par distillation, ces composés formant en effet souvent des mélanges azéotropiques.

La demande de brevet WO-A1-97/05089 décrit, entre autres, un procédé de purification d'hydro(chloro)fluoroalcanes (en particulier, le 1,1,1,3,3-pentafluoropropane ou HFC-245fa) de mélanges azéotropiques avec le fluorure d'hydrogène par une technique de distillation azéotropique comprenant deux étapes de distillation successives à des températures et à des pressions différentes.

Cette technique de distillation azéotropique présente cependant les désavantages de requérir une grande différence de température ou de pression entre les deux colonnes de façon à disposer d'un potentiel de séparation suffisant (différence de composition entre l'azéotrope à basse pression/température et l'azéotrope à haute pression/température) et d'engendrer un important débit de circulation entre les deux colonnes.

La séparation par extraction avec des solvants organiques de fluorure d'hydrogène d'avec ses mélanges avec certains hydro(chloro)fluoroalcanes spécifiques contenant 2 atomes de carbone a été proposé dans JP 49/045842, JP 09/110738, EP 601373 et EP 588676.

La demande de brevet WO-A1-97/13719 divulgue un procédé de séparation et de récupération de fluorure d'hydrogène de ses mélanges (azéotropiques) avec, entre autres, des hydrofluoroalcanes contenant de 1 à 6 atomes de carbone (en particulier, le HFC-245fa). Le mélange est mis en contact avec une solution de fluorure de métal alcalin (en particulier, du fluorure de potassium ou de césium) et la phase organique est séparée de la phase contenant le fluorure d'hydrogène et le fluorure de métal alcalin.

Par ce procédé connu, on peut craindre la contamination de la phase organique par le fluorure de potassium ou de césium et le risque de décomposition des hydrofluoroalcanes que cette contamination pourrait entraîner. D'autre part, ces fluorures de métaux alcalins, et plus particulièrement le fluorure de césium, sont très coûteux.

La présente invention a pour objet de fournir un procédé pour la séparation du fluorure d'hydrogène de ses mélanges avec un hydrofluoroalcane contenant de 3 à 6 atomes de carbone qui ne présente pas les inconvénients des procédés précités.

A cet effet, l'invention concerne un procédé de séparation du fluorure d'hydrogène de ses mélanges avec au moins un hydrofluoroalcane contenant de 3 à 6 atomes de carbone, selon lequel la séparation est effectuée par extraction au moyen d'au moins un solvant organique.

Le solvant organique peut être un composé halogéné ou non-halogéné.

A titre d'exemple de solvant organique non-halogéné, on peut citer les hydrocarbures contenant de 5 à 10 atomes de carbone, en particulier le n-pentane, le n-hexane, le n-heptane et le n-octane.

Des exemples de solvants organiques halogénés sont le chloroforme, le trichloréthylène, le tétrachloréthylène, le tétrachlorométhane, le 1,2-dichloréthane, le 1,1,1-trichloréthane, le 1,1,2-trichloréthane, le 1,1-dichloro-1-fluoroéthane (HCFC-141b), le 1,1,1- ou le 1,1,2-trifluorotrichloréthane, le 1,2,3-trichloropropane, des hydrocarbures perfluorés, le bromobenzène, l'o-dichlorobenzène, le p-chlorotoluène, le p-chlorotrifluorobenzène et le 1,2-dichloro-4-trifluorobenzène, de même que des mélanges de ces composés.

En particulier, lorsque le procédé est appliqué à la séparation du mélange fluorure d'hydrogène - 1,1,1,3,3-pentafluorobutane (HFC-365mfc), les solvants d'extraction préférés sont le bromobenzène, l'o-dichlorobenzène et le tétrachloréthylène. Le bromobenzène et l'o-dichlorobenzène sont particulièrement préférés.

Par hydrofluoroalcane contenant de 3 à 6 atomes de carbone, on entend désigner les hydrofluoroalcanes répondant à la formule générale CₐH_{(2a+2)-b}F_{b} dans laquelle a = 3 à 6 et b = 1 à 2a+1. Les hydrofluoroalcanes répondant à la formule générale CₐH_{(2a+2)-b}F_{b} dans laquelle a = 3 à 4 et b = 5 à 2a+1 sont préférés.

A titre d'exemple d'hydrofluoroalcanes contenant de 3 à 6 atomes de carbone séparables de leurs mélanges avec le fluorure d'hydrogène par le procédé selon l'invention, on peut citer, le 1,1,1,3,3-pentafluoropropane (HFC-245fa), le 1,1,2,2,3-pentafluoropropane (HFC-245ca), le 1,1,1,2,3-pentafluoropropane (HFC-245eb), le 1,1,1,3,3,3-hexafluoropropane (HFC-236fa), le 1,1,1,2,3,3-hexafluoropropane (HFC-236ea), le 1,1,1,2,3,3,3-heptafluoropropane (HFC-227ea), le 1,1,1,3,3-pentafluoro-2-méthylpropane (HFC-365mps), le 1,1,1,3,3-pentafluorobutane (HFC-365mfc), le 1,1,1,4,4,4-hexafluorobutane (HFC-356mff) et le 1,1,1,2,3,4,4,5,5,5-décafluoropentane (HFC-43-10mee). Les composés contenant de 3 à 4 atomes de carbone sont préférés. Le 1,1,1,3,3-pentafluoropropane (HFC-245fa) et le 1,1,1,3,3-pentafluorobutane (HFC-365mfc) sont particulièrement préférés.

Le rapport pondéral entre le fluorure d'hydrogène et l'hydrofluoroalcane dépend de la quantité de fluorure d'hydrogène mis en oeuvre lors de l'étape d'hydrofluoration du procédé de synthèse de l'hydrofluoroalcane. En général, le fluorure d'hydrogène est en excès par rapport à l'hydrofluoroalcane.

De manière préférée, on effectue l'extraction sur un mélange ayant un rapport pondéral fluorure d'hydrogène/hydrofluoroalcane proche de la composition azéotropique. Si le mélange initial de fluorure d'hydrogène et d'hydrofluoroalcane s'écarte de la composition azéotropique, il peut être avantageux d'effectuer au préalable une distillation pour séparer l'azéotrope HF/HFC du composé en excès (HF ou HFC). La composition azéotropique est ensuite soumise à l'extraction.

Dans le procédé de séparation selon l'invention, le rapport pondéral entre le solvant organique et le mélange de fluorure d'hydrogène et d'hydrofluoroalcane est généralement d'au moins 0.1. De préférence, on travaille avec un rapport pondéral d'au moins 0.2. Le rapport pondéral entre le solvant organique et le mélange de fluorure d'hydrogène et d'hydrofluoroalcane ne dépasse en général pas 10. De préférence, il ne dépasse pas 5.

La température à laquelle l'extraction est effectuée est généralement d'au moins -25 °C. De préférence, elle est d'environ -10°C. La température ne dépasse en général pas 40°C. De préférence, elle ne dépasse pas 30°C.

Le procédé selon l'invention est réalisé à une pression suffisante pour maintenir le mélange à l'état liquide. Il peut être réalisé sous la pression autogène du mélange; dans ce cas la pression est généralement inférieure à 3 bar. Alternativement, il peut être réalisé à une pression supérieure à la pression autogène du mélange en admettant un gaz inerte. Dans ce cas, la pression totale sera généralement inférieure à 10 bar; de manière préférée, la pression mise en oeuvre sera inférieure à 3 bar mais supérieure à 1 bar. A titre de gaz inerte, on utilisera toute substance gazeuse ne réagissant pas substantiellement dans les conditions d'extraction telle que l'azote, le chlorure d'hydrogène, l'argon ou un mélange de ceux-ci. De préférence, on utilisera l'azote.

La mise en contact du mélange de fluorure d'hydrogène et d'hydrofluoroalcane avec le solvant organique d'extraction est réalisée en une ou plusieurs étapes, au moyen de n'importe quel dispositif conventionnel d'extraction liquide-liquide, par exemple par mise en contact intime au moyen d'un mélangeur statique, d'un réacteur agité, d'un extracteur à disques rotatifs, d'un extracteur à centrifugation ou d'une colonne à plateaux perforés, fonctionnant soit à contre-courant, soit à co-courant. De préférence, la mise en contact est effectuée au sein d'un réacteur agité. L'extraction peut être menée en continu ou en discontinu. De préférence, elle est menée en continu.

Après extraction, on sépare une phase organique enrichie en hydrofluoroalcane d'une phase enrichie en fluorure d'hydrogène (appelée ci-après phase HF). Cette séparation peut être réalisée simplement par décantation, mais on peut aussi mettre en oeuvre tout autre dispositif classique de séparation de phases, tel qu'une centrifugation ou une séparation par hydrocyclone. La séparation par décantation est préférée.

La phase organique comprend principalement le solvant d'extraction enrichi en hydrofluoroalcane, mais peut aussi contenir une certaine quantité de fluorure d'hydrogène. Sa composition correspond le plus souvent à la composition d'équilibre, déterminée par les coefficients de partage des différents composés entre le fluorure d'hydrogène et le solvant d'extraction.

L'hydrofluoroalcane peut aisément être séparé des autres constituants de la phase organique par une technique de séparation classique telle qu'une distillation. L'hydrofluoroalcane peut ensuite être traité par voie humide pour éliminer les dernières traces d'acidité et/ou adsorbé sur charbon actif et/ou désacidifié sur zéolithe ou alumine. Le solvant peut être recyclé à l'étape d'extraction.

La phase HF contient principalement du fluorure d'hydrogène appauvri en hydrofluoroalcane, mais peut aussi contenir une quantité non négligeable de solvant d'extraction.

Lorsque le solvant d'extraction est inerte dans les conditions de réaction, la phase HF peut être renvoyée telle quelle directement au réacteur d'hydrofluoration pour la production d'hydrofluoroalcane.

Selon un autre mode de mise en oeuvre du procédé, la phase HF peut être avantageusement soumise à un traitement approprié tel qu'une distillation permettant de récupérer du fluorure d'hydrogène pratiquement débarrassé de solvant en tête de colonne, qui est recyclé au réacteur, et du solvant en pied de colonne, qui est recyclé à l'étape d'extraction.

Les exemples qui suivent sont destinés à illustrer la présente invention sans toutefois en limiter la portée.

### Exemples 1 à 11

Dans un autoclave de 0.5 l en INOX 316 équipé d'un agitateur à pales, de deux tubes plongeants permettant de prélever des échantillons des deux phases liquides (au fond et en haut du réacteur) et d'un doigt de gant muni d'un thermocouple permettant de mesurer la température, on a introduit un mélange de 1,1,1,3,3-pentafluorobutane (HFC-365mfc) et de HF proche de la composition azéotropique et on l'a extrait au moyen d'un solvant. Le solvant utilisé ainsi que les quantités en poids de HFC-365mfc, de HF et de solvant mis en oeuvre sont repris dans le tableau I. L'autoclave est plongé dans un bain maintenu à température constante. L'extraction a été effectuée à -10°C sous une pression autogène c'est-à-dire légèrement inférieure à 1 bar. Le mélange a été agité pendant 1 heure (exemple 10), 4 heures (ex. 1 à 5 et 8) ou 24 heures (ex. 6, 7, 9 et 11), puis on l'a laissé décanter pendant au moins 1 heure.

Pour chaque exemple, l'efficacité de l'extraction a été évaluée après prélèvements dans chacune des deux phases liquides. Ces prélèvements de liquide ont été réalisés via un sas de 5 cm³ environ après avoir pressurisé l'autoclave sous 2 bar d'azote. Les échantillons de liquide prélevés dans la phase HF ont ensuite été détendus dans un récipient contenant une réserve d'éthanol. La teneur en HF a été dosée sur cette solution éthanolique diluée à l'eau (environ 100 fois) au moyen d'une électrode spécifique. Quant à la phase organique, sa composition a été déterminée par analyse chromatographique en phase vapeur après neutralisation du fluorure d'hydrogène à la chaux. Les résultats de ces analyses sont repris dans le tableau I.

**TABLEAU I**

| Ex. | Solvant | Composition de départ (g) | | | HFC/HF au départ (g/g) | Composition de la phase organique (% en poids) | | | HFC/HF dans la phase organique (g/g) |
|---|---|---|---|---|---|---|---|---|---|
| | | HFC | HF | solv. | | HFC | HF | solv. | |
| 1 | tétrachloréthylène | 130 | 93 | 102 | 1.4 | 11.8 | - | 88.2 | - |
| 2 | tétrachloréthylène | 101 | 93 | 100 | 1.1 | 9.3 | 0.1 | 90.6 | 93.0 |
| 3 | trichloréthylène | 101 | 116 | 100 | 0.9 | 34.2 | 0.4 | 65.4 | 85.5 |
| 4 | 1,2-dichloréthane | 98 | 102 | 99 | 1.0 | 39.3 | 2.8 | 57.9 | 14.0 |
| 5 | 1,1,2-trichloréthane | 96 | 106 | 99 | 0.9 | 39.0 | 1.1 | 59.9 | 35.5 |
| 6 | tétrachlorométhane | 96 | 92 | 95 | 1.0 | 34.2 | 0.8 | 65.0 | 42.8 |
| 7 | tétrachlorométhane | 100 | 93 | 103 | 1.1 | 29.7 | 0.4 | 69.9 | 74.3 |
| 8 | 1,2,3-trichloropropane | 106 | 109 | 103 | 1.0 | 32.3 | 1.9 | 65.8 | 17.0 |
| 9 | o-dichlorobenzène | 100 | 92 | 101 | 1.1 | 31.5 | 0.2 | 68.2 | 157.5 |
| 10 | bromobenzène | 101 | 102 | 152 | 1.0 | 21.7 | 0.2 | 78.1 | 108.5 |
| 11 | n-octane | 101 | 102 | 102 | 1.0 | 15.0 | 0.7 | 84.3 | 21.4 |

Les résultats du tableau I montrent que la concentration en 1,1,1,3,3-pentafluorobutane (HFC-365mfc) dans la phase organique varie entre environ 9% en poids dans le cas du tétrachloréthylène jusqu'à 30 à 40% en poids pour les autres solvants.

La concentration en fluorure d'hydrogène dans la phase organique est la plus basse dans le cas du tétrachloréthylène.

Les solvants avec lesquels on obtient un rapport HFC/HF dans la phase organique le plus élevé c'est-à-dire qui permettent d'extraire un maximum de 1,1,1,3,3-pentafluorobutane (HFC-365mfc) avec un minimum de fluorure d'hydrogène sont l'o-dichlorobenzène, le bromobenzène, le tétrachloréthylène, le trichloréthylène et le tétrachlorométhane.

### Exemples 12 et 13

Le même mode opératoire que pour les exemples 1 à 11 a été appliqué à l'extraction du mélange HF/1,1,1,3,3-pentafluoropropane (HFC-245fa) par le tétrachloréthylène et par l'o-dichlorobenzène mis à part que dans l'exemple 12, l'extraction a été effectuée à -12°C et le mélange a été agité pendant 15 heures, puis on l'a laissé décanter pendant 3 heures et dans l'exemple 13, l'extraction a été effectuée à -7°C et le mélange a été agité pendant 65 heures, puis on l'a laissé décanter pendant 3 heures.

Des prélèvements de liquide ont été réalisés comme pour les exemples 1 à 11 mis à part que les échantillons de liquide prélevés dans le sas ont été détendus dans un récipient contenant un mélange d'eau et de tétrachlorométhane. La composition de la phase organique présente dans le réacteur a été analysée. Les résultats de ces analyses sont repris dans le tableau II.

**TAEAU II**

| Ex. | Solvant | Composition de départ (g) | | | HFC/HF au départ (g/g) | Composition de la phase organique (*) (% en poids) | | | HFC/HF dans la phase organique (g/g) |
|---|---|---|---|---|---|---|---|---|---|
| | | HFC | HF | solv. | | HFC | HF | solv. | |
| 12 | tétrachloréthylène | 67.5 | 90.2 | 161.0 | 0.75 | 3.75 | 0.17 | 96.1 | 22.1 |
| 13 | o-dichlorobenzène | 101.1 | 102.3 | 128.7 | 0.99 | 9.5 | 0.2 | 90.3 | 47.5 |

| | | | | | | | | | |
|---|---|---|---|---|---|---|---|---|---|
| (*) présente dans le réacteur | | | | | | | | | |

## Revendications

1. Procédé de séparation de fluorure d'hydrogène de ses mélanges avec au moins un hydrofluoroalcane contenant de 3 à 6 atomes de carbone, autre que le 1,1,1,3,3-pentafluoropropane, selon lequel la séparation est effectuée par extraction au moyen d'au moins un solvant organique.

2. Procédé selon la revendication 1, dans lequel le solvant organique est un composé organique halogéné.

3. Procédé selon la revendication 1 ou 2, dans lequel le solvant organique est choisi parmi le chloroforme, le trichloréthylène, le tétrachloréthylène, le tétrachlorométhane, le 1,2-dichloréthane, le 1,1,1-trichloréthane, le 1,1,2-trichloréthane, le 1,1-dichloro-1-fluoroéthane (HCFC-141b), le 1,1,1- ou le 1,1,2-trifluorotrichloréthane, le 1,2,3-trichloropropane, les hydrocarbures perfluorés, le bromobenzène, l'o-dichlorobenzène, le p-chlorotoluène, le p-chlorotrifluorobenzène et le 1,2-dichloro-4-trifluorobenzène et des mélanges de ces composés.

4. Procédé selon l'une quelconque des revendications 1 à 3, dans lequel le solvant organique est choisi parmi le bromobenzène, l'o-dichlorobenzène et le tétrachloréthylène.

5. Procédé selon la revendication 1, dans lequel le solvant organique est un hydrocarbure contenant de 5 à 10 atomes de carbone.

6. Procédé selon l'une quelconque des revendications 1 à 5, dans lequel l'hydrofluoroalcane contenant de 3 à 6 atomes de carbone est choisi parmi le 1,1,1,3,3-pentafluorobutane.

7. Procédé de séparation de fluorure d'hydrogène de ses mélanges avec le 1,1,1,3,3-pentafluoropropane, selon lequel la séparation est effectuée par extraction au moyen d'au moins un solvant organique halogéné ou au moins un hydrocarbure contenant de 5 à 10 atomes de carbone à l'exception d'un procédé dans lequel le solvant organique halogéné est choisi dans le groupe constitué.
(a) d'un hydrofluorocarbone répondant à la formule générale
CₓF_{y}H_{z},
dans laquelle x est un nombre entier de 3 à 10, y est un nombre entier de 2 à 22 et z est un nombre entier de 0 à 6 ;
(b) d'une amine répondant à la formule générale
R₁R₂R₃N
dans laquelle R₁, R₂ et R₃ sont des groupements fluoroalkyle ayant chacun de 1 à 10 atomes de carbone, et R₁, R₂ et R₃ respectivement peuvent contenir au plus deux atomes d'hydrogène ;
(c) d'un éther répondant à la formule générale
R₄R₅O
dans laquelle R₄ et R₅ sont des groupements alkyle ayant chacun de 1 à 10 atomes de carbone et au moins un parmi R₄ et R₅ contient un atome de fluor ;
(d) d'un hydrochlorocarbone répondant à la formule générale
C₁ClₘHₙ,
dans laquelle 1 est un nombre entier de 3 à 10, m est un nombre entier de 2 à 22 et n est un nombre entier de 0 à 6, le trichloroéthylène et le perchloroéthylène.

8. Procédé selon la revendication 7 dans lequel le solvant organique halogéné est choisi parmi le 1,1-dichloro-1-fluoroéthane (HCFC-141b), le 1,1,1-ou le 1,1,2-trifluorotrichloréthane, le bromobenzène, le p-chlorotrifluorobenzène, le 1,2-dichloro-4-trifluorobenzène et des mélanges de ces composés.

9. Procédé selon l'une quelconque des revendications 1 à 8, dans lequel le rapport pondéral entre le solvant organique et le mélange de fluorure d'hydrogène et d'hydrofluoroalcane est compris entre 0.1 et 10.

10. Procédé selon l'une quelconque des revendications 1 à 9, dans lequel l'extraction est effectuée à une température comprise entre -25°C et 40°C.

11. Procédé selon l'une quelconque des revendications 1 à 10, dans lequel l'extraction est effectuée sous la pression autogène du mélange.

12. Procédé de préparation d'un hydrofluoroalcane contenant de 3 à 6 atomes de carbone selon lequel
(a) on soumet dans un réacteur d'hydrofluoration un précurseur chloré approprié de l'hydrofluoroalcane à une réaction avec du fluorure d'hydrogène
(b) on soumet un mélange de produits réactionnels issus de la réaction et contenant de l'hydrofluoroalcane et du fluorure d'hydrogène non-converti au procédé de séparation selon l'une quelconque des revendications 1 à 11, étant entendu que lorsque l'hydrofluoroalcane est le 1,1,1,3,3-pentafluoropropane, le solvant organique est choisi parmi les composés cités dans la revendication 7 ou 8
(c) on sépare une phase organique enrichie en hydrofluoroalcane d'une phase HF enrichie en fluorure d'hydrogène.

13. Procédé selon la revendication 12, dans lequel la phase HF contient du solvant organique et ladite phase HF est soumise à une distillation et ou récupère du fluorure d'hydrogène en tête de colonne que l'on recycle au réacteur d'hydrofluoration et on récupère du solvant organique en pied de la colonne que l'on recycle à l'étape d'extraction.

14. Procédé selon la revendication 12, dans lequel la phase HF est recyclée telle quelle directement au réacteur d'hydrofluoration.

## Claims

1. Process for the separation of hydrogen fluoride from its mixtures with at least one hydrofluoroalkane comprising from 3 to 6 carbon atoms, other than 1,1,1,3,3-pentafluoropropane, according to which the separation is carried out by extraction by means of at least one organic solvent.

2. Process according to Claim 1, in which the organic solvent is a halogenated organic compound.

3. Process according to Claim 1 or 2, in which the organic solvent is chosen from chloroform, trichloroethylene, tetrachloroethylene, tetrachloromethane, 1,2-dichloroethane, 1,1,1-trichloroethane, 1,1,2-trichloroethane, 1,1-dichloro-1-fluoroethane (HCFC-141b), 1,1,1- or 1,1,2-trifluorotrichloroethane, 1,2,3-trichloropropane, perfluorinated hydrocarbons, bromobenzene, o-dichlorobenzene, p-chlorotoluene, p-chloro-trifluorobenzene and 1,2-dichloro-4-trifluorobenzene and mixtures of these compounds.

4. Process according to any one of Claims 1 to 3, in which the organic solvent is chosen from bromobenzene, o-dichlorobenzene and tetrachloroethylene.

5. Process according to Claim 1, in which the organic solvent is a hydrocarbon comprising from 5 to 10 carbon atoms.

6. Process according to any one of Claims 1 to 5, in which the hydrofluoroalkane comprising from 3 to 6 carbon atoms is chosen from 1,1,1,3,3-pentafluorobutane.

7. Process for the separation of hydrogen fluoride from its mixtures with 1,1,1,3,3-pentafluoropropane, according to which the separation is carried out by extraction by means of at least one halogenated organic solvent or at least one hydrocarbon comprising from 5 to 10 carbon atoms, with the exception of a process in which the halogenated organic solvent is chosen from the group consisting
(a) of a hydrofluorocarbon corresponding to the general formula
CₓF_{y}H_{z}
in which x is an integer from 3 to 10, y is an integer from 2 to 22 and z is an integer from 0 to 6;
(b) of an amine corresponding to the general formula
R₁R₂R₃N
in which R₁, R₂ and R₃ are fluoroalkyl groups each having from 1 to 10 carbon atoms and R₁, R₂ and R₃ can respectively comprise at most two hydrogen atoms;
(c) of an ether corresponding to the general formula
R₄R₅O
in which R₄ and R₅ are alkyl groups each having from 1 to 10 carbon atoms and at least one from R₄ and R₅ comprises a fluorine atom;
(d) of a hydrochlorocarbon corresponding to the general formula
CₗClₘHₙ
in which 1 is an integer from 3 to 10, m is an integer from 2 to 22 and n is an integer from 0 to 6, trichloroethylene and perchloroethylene.

8. Process according to Claim 7, in which the halogenated organic solvent is chosen from 1,1-dichloro-1-fluoroethane (HCFC-141b), 1,1,1- or 1,1,2-trifluorotrichloroethane, bromobenzene, p-chlorotrifluorobenzene, 1,2-dichloro-4-trifluorobenzene and mixtures of these compounds.

9. Process according to any one of Claims 1 to 8, in which the ratio by weight of the organic solvent to the mixture of hydrogen fluoride and of hydrofluoroalkane is between 0.1 and 10.

10. Process according to any one of Claims 1 to 9, in which the extraction is carried out at a temperature of between -25°C and 40°C.

11. Process according to any one of Claims 1 to 10, in which the extraction is carried out under the autogenous pressure of the mixture.

12. Process for the preparation of a hydrofluoroalkane comprising from 3 to 6 carbon atoms, according to which
(a) an appropriate chlorinated precursor of the hydrofluoroalkane is reacted with hydrogen fluoride in a hydrofluorination reactor,
(b) a mixture of reaction products resulting from the reaction and comprising hydrofluoroalkane and unconverted hydrogen fluoride is subjected to the separation process according to any one of Claims 1 to 11, it being understood that, when the hydrofluoroalkane is 1,1,1,3,3-pentafluoropropane, the organic solvent is chosen from the compounds cited in Claim 7 or 8,
(c) an organic phase enriched in hydrofluoroalkane is separated from an HF phase enriched in hydrogen fluoride.

13. Process according to Claim 12, in which the HF phase comprises organic solvent and the said HF phase is distilled and hydrogen fluoride is recovered at the column head, which hydrogen fluoride is recycled to the hydrofluorination reactor, and organic solvent is recovered at the bottom of the column, which organic solvent is recycled to the extraction stage.

14. Process according to Claim 12, in which the HF phase is recycled as is directly to the hydrofluorination reactor.

## Patentansprüche

1. Verfahren zur Abtrennung von Fluorwasserstoff aus seinen Gemischen mit wenigstens einem Hydrofluoralkan mit einem Gehalt an 3 bis 6 Kohlenstoffatomen, außer 1,1,1,3,3-Pentafluorpropan, wonach die Abtrennung durch Extraktion mit wenigstens einem organischen Lösungsmittel vorgenommen wird.

2. Verfahren nach Anspruch 1, worin das organische Lösungsmittel eine halogenierte organische Verbindung ist.

3. Verfahren nach Anspruch 1 oder 2, worin das organische Lösungsmittel unter Chloroform, Trichlorethylen, Tetrachlorethylen, Tetrachlormethan, 1,2-Dichlorethan, 1,1,1-Trichlorethan, 1,1,2-Trichlorethan, 1,1-Dichlor-1-fluorethan (HCFC-141b), 1,1,1- oder 1,1,2-Trifluortrichlorethan, 1,2,3-Trichlorpropan, den perfluorierten Kohlenwasserstoffen, Brombenzol, o-Dichlorbenzol, p-Chlortoluol, p-Chlortrifluorbenzol und 1,2-Dichlor-4-trifluorbenzol und Gemischen dieser Verbindungen ausgewählt wird.

4. Verfahren nach einem der Ansprüche 1 bis 3, worin das organische Lösungsmittel unter Brombenzol, o-Dichlorbenzol und Tetrachlorethylen ausgewählt wird.

5. Verfahren nach Anspruch 1, worin das organische Lösungsmittel ein 5 bis 10 Kohlenstoffatome enthaltender Kohlenwasserstoff ist.

6. Verfahren nach einem der Ansprüche 1 bis 5, worin das 3 bis 6 Kohlenstoffatome enthaltende Hydrofluoralkan unter dem 1,1,1,3,3-Pentafluorbutan ausgewählt wird.

7. Verfahren zur Abtrennung von Fluorwasserstoff aus seinen Gemischen mit 1,1,1,3,3-Pentafluorpropan, wonach die Abtrennung durch Extraktion mit wenigstens einem halogenierten organischen Lösungsmittel oder mit einem wenigstens 5 bis 10 Kohlenstoffatome enthaltenden Kohlenwasserstoff ausgeführt wird, mit Ausnahme eines Verfahrens, worin das halogenierte organische Lösungsmittel aus der Gruppe ausgewählt wird, die aus
(a) einem Hydrofluorkohlenstoff entsprechend der allgemeinen Formel CₓFyH_{z}, worin x eine ganze Zahl von 3 bis 10 ist, y eine ganze Zahl von 2 bis 22 ist und z eine ganze Zahl von 0 bis 6 ist;
(b) einem Amin mit der allgemeinen Formel R₁R₂R₃N, worin R₁, R₂ und R₃ Fluoralkylgruppen mit einem Gehalt an jeweils 1 bis 10 Kohlenstoffatomen sind und R₁, R₂ bzw. R₃ höchstens zwei Wasserstoffatome enthalten können;
(c) einem Ether mit der allgemeinen Formel R₄R₅O, worin R₄ und R₅ Alkylgruppen mit jeweils 1 bis 10 Kohlenstoffatomen sind und wenigstens einer der Reste R₄ und R₅ ein Fluoratom enthält;
(d) einem Hydrochlorkohlenstoff mit der allgemeinen Formel CₗClₘHₙ, worin 1 eine ganze Zahl von 3 bis 10 ist, m eine ganze Zahl von 2 bis 22 ist und n eine ganze Zahl von 0 bis 6 ist, dem Trichlorethylen und dem Perchlorethylen
besteht.

8. Verfahren nach Anspruch 7, worin das halogenierte organische Lösungsmittel unter 1,1-Dichlor-1-fluorethan (HCFC-141b), 1,1,1- oder 1,1,2-Trifluortrichlorethan, Brombenzol, p-Chlortrifluorbenzol, 1,2-Dichlor-4-trifluorbenzol und den Gemischen dieser Verbindungen ausgewählt wird.

9. Verfahren nach einem der Ansprüche 1 bis 8, worin das Gewichtsverhältnis zwischen dem organischem Lösungsmittel und dem Gemisch aus Fluorwasserstoff und Hydrofluoralkan zwischen 0,1 und 10 beträgt.

10. Verfahren nach einem der Ansprüche 1 bis 9, worin die Extraktion bei einer Temperatur zwischen -25°C und 40°C ausgeführt wird.

11. Verfahren nach einem der Ansprüche 1 bis 10, worin die Extraktion unter dem autogenen Druck des Gemisches vorgenommen wird.

12. Verfahren zur Herstellung eines Hydrofluoralkans mit einem Gehalt an 3 bis 6 Kohlenstoffatomen, wonach man
(a) in einem Hydrofluorierungsreaktor einen entsprechenden chlorierten Vorläufer des Hydrofluoralkans einer Umsetzung mit Fluorwasserstoff unterwirft,
(b) ein aus der Umsetzung entstehendes Gemisch von Reaktionsprodukten mit einem Gehalt an Hydrofluoralkan und nicht umgewandeltem Fluorwasserstoff einem Trennverfahren nach einem der Ansprüche 1 bis 11 unterwirft, wobei dann, wenn das Hydrofluoralkan das 1,1,1,3,3-Pentafluorpropan ist, das organische Lösungsmittel unter den im Anspruch 7 oder 8 angeführten Verbindungen ausgewählt wird,
(c) eine an Hydrofluoralkan angereicherte organische Phase von einer an Fluorwasserstoff angereicherten HF-Phase abtrennt.

13. Verfahren nach Anspruch 12, worin die HF-Phase organisches Lösungsmittel enthält und diese HF-Phase einer Destillation unterworfen wird, bei der am Kopf der Kolonne Fluorwasserstoff gewonnen wird, der zum Hydrofluorierungsreaktor recycliert wird, und am Kolonnensumpf organisches Lösungsmittel gewonnen wird, das zur Extraktionsstufe recycliert wird.

14. Verfahren nach Anspruch 12, worin die HF-Phase so wie sie ist direkt zum Hydrofluorierungsreaktor recycliert wird.
